# FASCICULE DE BREVET EUROPEEN

(11) **EP 1 518 538 B1**
(45) Date de publication et mention de la délivrance du brevet: **29.12.2010**
(21) Numéro de dépôt: 04292304.5
(22) Date de dépôt: 27.09.2004
(51) Int. Cl.: A61K 8/87, A61K 8/85, A61Q 5/06

(54) **Composition cosmétique sous forme d'aérosol contenant l'association polyester sulfonique dispersible dans l'eau/polyuréthane non-associatif**
Kosmetische Aerosol Zusammensetzung enthaltend einen sulfonierten Polyester und ein nicht-assoziatives Polyurethan
Cosmetic composition in aerosol form comprising the association of a water-dispersible sulfopolyester and of a non-associative polyurethane

(30) Priorité: 26.09.2003 FR 0311319
(43) Date de publication de la demande: 30.03.2005
(73) Titulaire: L'Oréal, 75008 Paris (FR)
(72) Inventeur: Pataut, Francoise, 75017 Paris (FR); Breton, Marie-Laure, 75011 Paris (FR); Bremenson, Jean-Luc, 92800 Puteaux (FR)
(74) Mandataire: Dossmann, Gérard

(56) Documents cités:
- EP-A- 0 993 824
- EP-A- 1 013 256
- WO-A-95/33436
- FR-A- 2 774 898
- FR-A- 2 831 437

## Description

La présente invention concerne un dispositif aérosol comprenant une composition de traitement cosmétique des matières kératiniques, en particulier des cheveux, une utilisation de cette composition conditonnée dans le dispositif aérosol pour la mise en forme et/ou le maintien de la coiffure, ainsi qu'un procédé de traitement cosmétique le mettant en ouvre.

Dans le domaine du coiffage et/ou de la fixation, les compositions capillaires se présentent généralement comme des compositions à pulvériser essentiellement constituées d'une solution alcoolique et d'un ou de plusieurs matériaux fixants en mélange avec divers adjuvants cosmétiques. Cette solution est généralement conditionnée soit dans un dispositif aérosol approprié mis sous pression à l'aide d'un agent propulseur, soit dans un flacon pompe.

On connaît de nombreux dispositifs aérosols destinés à fixer la coiffure, ces dispositifs contenant d'une part une phase liquide (ou jus) et d'autre part un agent propulseur. Ce dernier a pour fonction d'assurer une pression permettant la pulvérisation de la phase liquide, et son application sur les cheveux sous forme d'un nuage de gouttelettes dispersées.

Pour des raisons essentiellement écologiques, on cherche à réduire la quantité de composés organiques volatils (ou COV) présents dans la composition. Pour diminuer la quantité de COV et obtenir un dispositif aérosol à faible COV, les solvants organiques, comme l'éthanol, sont remplacés en partie par l'eau.

La phase liquide contient notamment des matériaux fixants, par exemples anioniques. Cependant, leur association avec une grande quantité d'eau implique une augmentation de la viscosité de la phase liquide et une dégradation de la restitution du produit, par exemple obtention d'un spray blanc, qui sont dues à cette diminution de la quantité d'alcools dans le jus aérosol.

La Demanderesse a découvert de manière surprenante qu'une association particulière d'un polyester sulfonique dispersible dans l'eau et d'un polyuréthane non-associatif particulier permettait d'obtenir une mise en forme et/ou un excellent maintien de la coiffure en conférant une fixation forte, voire très forte.

Lorsqu'une composition comprenant une telle association est conditionnée sous forme aérosol et si la concentration de polymère(s) est élevée, une bonne restitution de la composition est obtenue car la viscosité de la phase liquide reste faible.

L'association du polyester sulfonique dispersible dans l'eau et d'un polyuréthane non-associatif particulier permet également une totale élimination du produit au cours d'un shampoing.

L'invention a donc pour objet un dispositif aérosol comprenant une composition de traitement cosmétique des matières kératiniques, en particulier des cheveux, qui comprend dans un milieu aqueux cosmétiquement acceptable, une telle association, et au moins un agent propulseur. Un autre objet de la présente invention consiste en une utilisation d'une composition conditionnée dans ledit dispositif aérosol pour la mise en forme et le maintien de la coiffure.

Un autre objet encore est un procédé de traitement cosmétique mettant en ouvre la composition conditionnée dans ledit dispositif aérosol.

D'autres objets, caractéristiques, aspects et avantages de l'invention apparaîtront encore plus clairement à la lecture de la description et des divers exemples qui suivent.

Selon l'invention, la composition de traitement cosmétique des matières kératiniques, en particulier des cheveux, comprend dans un milieu aqueux cosmétiquement acceptable, au moins un polyester sulfonique dispersible dans l'eau, et au moins un polyuréthane non associatif.

Par milieu cosmétiquement acceptable, on entend un milieu compatible avec les cheveux, mais aussi d'odeur, d'aspect et de toucher agréables.

Par polyester sulfonique hydrodispersible, on entend tout polyester sulfonique présentant une aptitude à former une dispersion, c'est-à-dire un système biphasique où la première phase est formée de particules finement divisées et distribuées uniformément dans la seconde phase qui est la phase continue.

Par polyester sulfonique, on entend des copolyesters obtenus par polycondensation d'au moins un acide dicarboxylique, de préférence aromatique, et plus particulièrement différent de l'acide téréphtalique ou d'un de ses esters ou un de ses sels, d'au moins un diol et d'au moins un composé difonctionnel sulfoaryldicarboxylique substitué sur le noyau aromatique par un groupe -SO₃M dans lequel M représente un atome d'hydrogène ou un ion métallique tel que Na⁺, Li⁺ ou K⁺.

Le polyester sulfonique dispersible dans l'eau est obtenu à parti d'acide isophtalique éventuellement sous forme d'ester et d'acide sulfoisophtalique éventuellement sous forme de sel à titre de seuls acides dicarboxyliques présents et de diéthylèneglycol.

Les diols utilisés dans la synthèse des polyesters sulfoniques selon l'invention sont par exemple le diéthylèneglycol, le dipropylèneglycol, le tripropylèneglycol, le polyéthylèneglycol, le butane-(1,4)-diol, le 1,4-cyclohexaneméthanol.

Les polyesters sulfoniques dispersibles dans l'eau présentent généralement une masse moléculaire moyenne en poids comprise entre environ 1 000 et 60 000, et de préférence de 4 000 à 20 000, telle que déterminée par chromatographie par perméation de gel (ou GPC).

La température de transition vitreuse de ces polyesters sulfoniques est généralement comprise dans l'intervalle allant de 10 °C à 100 °C, de préférence de 25 à 60 °C.

Ils sont décrits plus en détail dans les demandes de brevet US 3 734 874, US 3 779 993, US 4 119 680, US 4 300 580, US 4 973 656, US 5 660 816, US 5 662 893, et US 5 674 479.

Les polyesters sulfoniques utilisés de préférence dans l'invention comprennent au moins des motifs dérivés d'acide isophtalique seul en tant qu'acide dicarboxylique, de sel d'acide sulfoaryle-dicarboxylique et de diéthylèneglycol, et éventuellement d'un autre diol, et plus particulièrement les polyesters sulfoniques utilisés dans l'invention sont obtenus à partir d'acide isophtalique seul en tant qu'acide dicarboxylique, de sel de sodium de l'acide sulfoisophtalique, de diéthylèneglycol et de 1,4-cyclohexaneméthanol.

A titre d'exemples de polyester sulfonique, on peut notamment citer ceux connus sous le nom INCI Diglycol/CHDM/Isophtalates/SIP, et vendus sous les dénominations commerciales EASTMAN AQ^{®}, par la société Eastman Chemical, et plus particulièrement celui vendu sous la dénomination commerciale EASTMAN AQ 48^{®}.

Par polyuréthane non associatif, on entend au sens de la présente invention, des polycondensats comprenant au moins une séquence polyuréthane et ne comprenant pas, dans leur structure, de chaîne alkyle ou alcényle, terminale ou pendante, comportant plus de 10 atomes de carbone. Ils sont décrits en particulier dans les brevets EP 0 751 162, EP 0 637 600, FR 2 743 297 et EP 0 648 485 dont la Demanderesse est titulaire, ainsi que les brevets EP 0 656 021 ou WO 94/03510 de la Société BASF et EP 0 619 111 de la Société National Starch.

Les polyuréthanes non associatifs utilisés conformément à l'invention peuvent être solubles dans le milieu aqueux cosmétiquement acceptable, notamment après neutralisation par une base organique ou minérale, ou encore former une dispersion dans ce milieu. La dispersion peut comprendre alors au moins 0,05 % de tensioactif permettant la mise en dispersion et le maintien en dispersion du polyuréthane non associatif.

Selon l'invention, on peut utiliser tout type de tensioactif dans ladite dispersion, mais de préférence un tensioactif non ionique. La taille moyenne des particules du polyuréthane non associatif dans la dispersion est de préférence comprise entre 0,1 et 1 micromètre.

Les polyuréthanes non associatifs utilisés dans l'invention comprennent un motif répétitif de base répondant à la formule générale (I):

-O-B-O-CO-NH-R-NH-CO- (1)

dans laquelle:
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes hydrocarbonés aliphatiques en C₁ à C₂₀, cycloaliphatiques en C₃ à C₂₀ et aromatiques en C₆ à C₂₀, comme par exemple les groupes alkylène en C₁ à C₂₀, arylène en C₆ à C₂₀, cycloalkylène en C₃ à C₂₀, ou leurs combinaisons, ces groupes étant substitués ou non.

Le groupe R est avantageusement choisi parmi les groupes répondant aux formules suivantes : 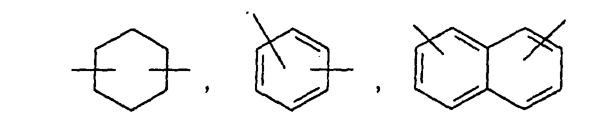 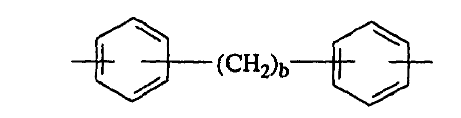 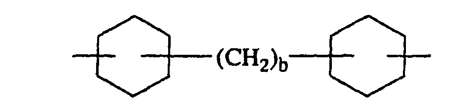 -(CH₂)_{c}-
dans lesquelles b est un nombre entier compris entre 0 et 3, et c un nombre entier compris entre 1 et 20, de préférence compris entre 2 et 12.

En particulier, le groupe R est choisi parmi les groupes hexaméthylène, 4,4'-biphénylèneméthane, 2,4- et/ou 2,6-tolylène, 1,5-naphtylène, p-phénylène, méthylène- 4,4-bis-cyclohexyle et le groupe divalent dérivé de l'isophorone.

Le polyuréthane non associatif utilisé dans la présente invention comprend en outre au moins une séquence polysiloxane dont le motif répétitif de base répond par exemple à la formule générale (II) :

-O-P-O-CO-NH-R-NH-CO- (II)

dans laquelle :
- P est un segment polysiloxanique, et
- R est un groupe divalent choisi parmi les groupes hydrocarbonés aliphatiques en C₁ à C₂₀, cycloaliphatiques en C₃ à C₂₀ et aromatiques en C₆ à C₂₀, comme par exemple les groupes alkylène en C₁ à C₂₀, arylène en C₆ à C₂₀, cycloalkylène en C₃ à C₂₀, ou leurs combinaisons, ces groupes étant substitués ou non.

Avantageusement, le segment polysiloxanique P répond à la formule générale (III) ci-après : 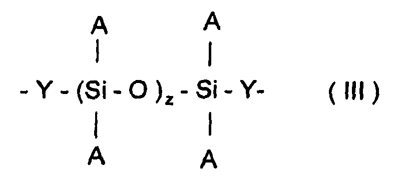 dans laquelle:
- les groupes A, qui peuvent être identiques ou différents, sont choisis parmi d'une part les groupes hydrocarbonés monovalents en C₁ à C₂₀ exempts ou substantiellement exempts d'insaturation éthylénique et, d'autre part, les groupes aromatiques,
- Y représente un groupe hydrocarboné divalent, et
- z représente un nombre entier, choisi de telle sorte que la masse moléculaire moyenne en poids du segment polysiloxane soit comprise entre 300 et 10 000.

De préférence, le groupe divalent Y est choisi parmi les groupes alkylène de formule -(CH₂)ₐ-, dans laquelle a représente un nombre entier pouvant être compris entre 1 et 10.

Les groupes A peuvent être choisis parmi les groupes alkyle en C₁₋₈, en particulier les groupes méthyle, éthyle, propyle, isopropyle, butyle, pentyle, hexyle, octyle ; les groupes cycloalkyle en C₃₋₈, en particulier le groupe cyclohexyle ; les groupes aryle en C₆₋₁₀, notamment phényle ; les groupes arylalkyle en C₇₋₁₀, notamment benzyle et phényléthyle, ainsi que les groupes tolyle et xylyle.

A titre d'exemples de polyuréthane non associatif, on peut notamment citer le copolymère acide diméthylolpropioniquelisophorone-diisocyanate/néopentylglycol/poly-esterdiols/diamine siliconée (connu aussi sous le nom de polyuréthane-6, appellation INCI) vendu sous la marque Luviset^{®} Si PUR A par la société BASF.

Les polyuréthanes non associatifs sont notamment présents dans la composition selon l'invention, en une quantité de 0,05 à 20 % en poids, de préférence de 0,1 à 10 % en poids, et mieux encore de 1 à 8 % en poids par rapport au poids total de la composition de traitement cosmétique des matières kératiniques.

Le ou les polyuréthane(s) utilisé(s) selon l'invention est (sont), de préférence, soluble(s) dans le milieu.

Le polyester sulfonique est présent dans la composition selon l'invention, en une quantité de 0,1 à 40 % en poids, de préférence de 1 à 30 %, et plus particulièrement de 5 à 25 % en poids par rapport au poids total de la composition de traitement cosmétique des matières kératiniques.

Le milieu aqueux cosmétiquement acceptable peut être constitué uniquement par de l'eau ou par un mélange d'eau et d'un ou plusieurs solvants cosmétiquement acceptables tels qu'un alcool inférieur en C₁-C₄, comme l'éthanol, l'isopropanol, le tertio-butanol, le n-butanol ; les alkylénepolyols comme le propylèneglycol ; les éthers de polyols ; et leurs mélanges. De préférence, le solvant additionnel est de l'éthanol.

La quantité d'eau présente dans les compositions de l'invention peut varier de préférence entre 15 et 95%, mieux encore entre 20 et 90%, et encore plus préférentiellement entre 25 et 80% en poids par rapport au poids total de la composition.

La composition selon l'invention peut comprendre en outre au moins un additif classique bien connu dans la technique, tel que d'autres polymères cationiques, amphotères, zwitteroniques, anioniques ou non ioniques différents de ceux décrits ci-dessus, des agents épaississants, des plastifiants, des nacrants, des opacifiants, des filtres UV, des sucres, des parfums, des huiles minérales, végétales et/ou synthétiques, des esters d'acides gras, des colorants, des silicones volatiles ou non, organomodifiées ou non, cycliques ou acycliques, ramifiées ou non, des particules minérales ou organiques, naturelles ou synthétiques, des conservateurs et des agents de stabilisation du pH.

L'homme du métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions de traitement cosmétique des matières kératiniques conditionnées dans le dispositif aérosol peuvent se présenter sous forme d'une mousse, d'un gel, d'un spray ou d'une laque, et être utilisées en application rincée ou non.

Les compositions conditionnées dans le dispositif aérosol peuvent être utilisées en tant que produits de mise en forme et/ou de maintien de la coiffure, compositions de soin de cheveux, shampoings, compositions de conditionnement des cheveux, telles que des compositions destinées à apporter de la douceur aux cheveux, ou encore des compositions de maquillage des cheveux. De préférence, il s'agit de compositions de mise en forme et/ou de maintien de la coiffure.

Les compositions selon l'invention sont conditionnées dans un dispositif aérosol usuel en cosmétique. Dans un tel cas, on peut utiliser au moins un quelconque agent propulseur bien connu dans la technique tel que les gaz hydrocarbonés comme les alcanes en C₃₋₅, par exemple, le propane, le n-butane, l'isobutane ; les gaz fluorés ; l'azote, l'air et le dioxyde de carbone ; le diméthyléther ; et leurs mélanges.

De préférence, l'agent propulseur est choisi parmi le diméthyléther, les gaz hydrocarbonés et leurs mélanges.

Le ou les agent(s) propulseur(s) peuvent utilisés en une quantité allant de 5 à 80 % en poids, de préférence 5 à 60 % en poids, et mieux encore de 5 à 40 % en poids par rapport au poids total de la composition de traitement cosmétique des matières kératiniquès.

La présente invention concerne également un procédé de traitement cosmétique des cheveux, par exemple de coiffage, qui consiste à appliquer une quantité efficace d'une composition tel que conditionnée dans un dispositif aérosol décrit ci-dessus, sur les cheveux, à rincer ou non après un éventuel temps de pose.

L'exemple suivant est donné à titre illustratif de la présente invention.

### EXEMPLE

On prépare une composition de coiffage sous forme de spray aérosol à 55 % en poids de composés organiques volatils, à partir des ingrédients suivants. Les quantités sont indiquées en % en poids :
- Polyester sulfonique : Eastman AQ 48® vendu 3 % MA^{*} par Eastman Chemical
- Polyuréthane fixant non associatif : Luviset® Si 4 % MA^{*} PUR vendu par BASF
- Ethanol 20 %
- Diméthyléther 35 %
- Eau 38 % ^{*}MA = matières actives

On pulvérise ce produit de coiffage sur la chevelure et on met en forme la coiffure. On obtient un bon maintien de la coiffure.

## Revendications

1. Dispositif aérosol comprenant une
Composition de traitement cosmétique des matières kératiniques, qui comprend dans un milieu aqueux cosmétiquement acceptable, au moins un polyester sulfonique dispersible dans l'eau, obtenu à partir d'acide isophtalique éventuellement sous forme d'ester et d'acide sulfoisophtalique éventuellement sous forme de sel à titre de seuls acides dicarboxyliques présents et de diéthylèneglycol et au moins un polyuréthane non-associatif et au moins un agent propulseur, étant sous forme libre ou bien neutralisées partiellement ou totalement par une base minérale ou organique, et
- R est un groupe divalent choisi parmi les groupes hydrocarbonés aliphatiques en C₁ à C₂₀, cycloaliphatiques en C₃ à C₂₀ et aromatiques en C₆ à C₂₀, ou leurs combinaisons, ces groupes étant substitués ou non,
et comprenant en outre un motif répétitif de base répondant à la formule générale (II) :
-O-F-O-CO-NH-R-NH-CO- dans laquelle : (II)
- P est un segment polysiloxanique, et
- R est un groupe divalent choisi parmi les groupes hydrocarbonés aliphatiques en C₁ à C₂₀, cycloaliphatiques en C₃ à C₂₀ et aromatiques en C₆ à C₂₀, ou leurs combinaisons, ces groupes étant substitués ou non.

2. Dispositif aérosol
selon la revendication 1, **caractérisé en ce que** le polyester sulfonique est obtenu à partir d'acide isophtalique ou d'un de ses esters, d'un sel de l'acide sulfoisophtalique à titre de seuls acides dicarboxyliques, de diéthylèneglycol.

3. Dispositif aérosol
selon la revendication 2, **caractérisé en ce que** le polyester sulfonique est obtenu à partir d'acide isophtalique, du sel de sodium de l'acide sulfoisophtalique à titre de seuls acides dicarboxyliques, de diéthyléneglycol.

4. Dispositif aérosol
selon l'une quelconque des revendications 1 à 3, **caractérisé en ce que** le polyester sulfonique est obtenu à partir d'acide isophtalique, de sel de sodium de l'acide sulfoisophtalique à titre de seuls acides dicarboxyliques, de diéthylèneglycol et d'un autre diol.

5. Dispositif aérosol
selon la revendication 4, **caractérisé en ce que** le polyester sulfonique est obtenu à partir d'acide isophtalique, de sel de sodium de l'acide sulfoisophtalique à titre de seuls acides dicarboxyliques, de diéthylèneglycol et de 1,4-cyclohexaneméthanol.
ledit polyuréthane non associatif comprenant un motif répétitif de base répondant à la formule générale (I) :
-O-B-O-CO-NH-R-NH-CO- (I)
dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques

6. Dispositif aérosol
selon l'une quelconque des revendications 1 à 5, **caractérisé en ce que** le polyuréthane non associatif est un copolymère acide diméthylol-propionique/isophorone-diisocyanate/néopentylglycol/polyester-diols/diamine siliconée.

7. Dispositif aérosol
selon l'une quelconque des revendications 1 à 6, **caractérisé en ce que** le polyuréthane non associatif est présent en une quantité de 0,05 à 20 % en poids, de préférence de 0,10 à 10 % en poids par rapport au poids total de la composition.

8. Dispositif aérosol
selon l'une quelconque des revendications 1 à 7, **caractérisé en ce que** le polyester sulfonique est présent en une quantité de 0,1 à 40 % en poids, de préférence de 1 à 30 %, et plus particulièrement de 5 à 25 % en poids par rapport au poids total de la composition.

9. Dispositif aérosol
selon l'une quelconque des revendications précédentes, **caractérisé en ce que** le milieu aqueux cosmétiquement acceptable est constitué uniquement par de l'eau ou par un mélange d'eau et d'un ou plusieurs solvants cosmétiquement acceptables.

10. Dispostif aérosol selon la revendication 9, **caractérisé en ce que** le solvant cosmétiquement acceptable est choisi parmi les alcools inférieurs en C₁-C₄, les alkylènepolyols, les éthers de polyols et leurs mélanges.

11. Dispositif aérosol kératiniques selon la revendication 9 ou 10, **caractérisé en ce que** le solvant cosmétiquement acceptable est choisi parmi l'éthanol, l'isopropanol, le tertio-butanol, le n-butanol et le propylèneglycol.

12. Dispositif aérosol
selon la revendication 11, **caractérisé en ce que** le solvant cosmétiquement acceptable est l'éthanol.

13. Dispositif aérosol selon l'une quelconque des revendications précédentes, **caractérisé en ce que** l'eau est présente une quantité variant entre 15
et 95%, de préférence entre 20 et 90% en poids par rapport au poids total de la composition.

14. Dispositif aérosol
selon l'une quelconque des revendications précédentes, **caractérisé en ce qu'**elle comprend en outre au moins un additif choisi parmi d'autres polymères cationiques, amphotères, zwitteroniques, anioniques ou non ioniques, différents de ceux tels que définis dans l'une quelconque des revendications 1 à 13, des agents épaississants, des plastifiants, des nacrants, des opacifiants, des filtres UV, des sucres, des parfums, des huiles minérales, végétales et/ou synthétiques, des esters d'acides gras, des colorants, des silicones volatiles ou non, organomodifiées ou non, cycliques ou acycliques, ramifiées ou non, des particules minérales ou organiques, naturelles ou synthétiques, des conservateurs et des agents de stabilisation du pH.

15. Dispositif aérosol
selon l'une quelconque des revendications précédentes, **caractérisé en ce qu'**elle se présente sous la forme d'une mousse, d'un gel, d'un spray ou d'une laque.

16. Dispositif aérosol selon l'une quelconque des revendication cation précedentes ce que l'agent propulseur est choisi parmi les gaz hydrocarbonés, les gaz fluorés, l'azote, l'air, le dioxyde de carbone, le diméthyléther et leurs mélanges.

17. Dispositif aérosol selon la revendication 16, **caractérisé en ce que** l'agent propulseur est choisi parmi le diméthyléther, les gaz hydrocarbonés et leurs mélanges.

18. Dispositif aérosol selon l'une quelconque des revendications 1 à 17, **caractérisé en ce que** l'agent propulseur est présent en une quantité de 5 à 80 % en poids, de préférence de 5 à 60 % en poids par rapport au poids total de la composition.

19. Utilisation de la composition de traitement cosmétique des matières kératiniques conditionnée dans un dispositif aérosol tel que défini dans les revendications précédentes, pour la mise en forme et/ou le maintien de la coiffure. >

20. Procédé de traitement cosmétique des cheveux, **caractérisé en ce que** l'on applique sur les cheveux la composition de traitement cosmétique conditionnée dans le dispotif aérosol selon l'une quelconque des revendications 1 à 18.

## Claims

1. Aerosol device comprising a cosmetic composition for treating keratin materials, which comprises, in a cosmetically acceptable aqueous medium, at least one water-dispersible sulphonic polyester obtained from isophthalic acid optionally in ester form and from sulphoisophthalic acid optionally in salt form, as the only dicarboxylic acids present, and from diethylene glycol, and at least one non-associative polyurethane and at least one propellant, said non-associative polyurethane comprising a repeating base unit corresponding to the general formula (I):
-O-B-O-CO-NH-R-NH-CO- (I)
in which:
- B is a divalent C₁ to C₃₀ hydrocarbon-based group, this group being optionally substituted with a group comprising one or more carboxylic acid functions and/or one or more sulphonic acid functions, the said carboxylic acid and/or sulphonic acid functions being in free form or partially or totally neutralized with a mineral or organic base, and
- R is a divalent group chosen from C₁ to C₂₀ aliphatic hydrocarbon-based groups, C₃ to C₂₀ cycloaliphatic groups and C₆ to C₂₀ aromatic groups, or combinations thereof, these groups being substituted or unsubstituted,
and also comprising a repeating base unit corresponding to the general formula (II):
-O-P-O-CO-NH-R-NH-CO- (II)
in which:
- P is a polysiloxane segment, and
- R is a divalent group chosen from C₁ to C₂₀ aliphatic hydrocarbon-based groups, C₃ to C₂₀ cycloaliphatic groups and C₆ to C₂₀ aromatic groups, or combinations thereof, these groups being substituted or unsubstituted.

2. Aerosol device according to Claim 1, **characterized in that** the sulphonic polyester is obtained from isophthalic acid or an ester thereof and from a salt of sulphoisophthalic acid, as the only dicarboxylic acids, and from diethylene glycol.

3. Aerosol device according to Claim 2, **characterized in that** the sulphonic polyester is obtained from isophthalic acid and from the sodium salt of sulphoisophthalic acid, as the only dicarboxylic acids, and from diethylene glycol.

4. Aerosol device according to any one of Claims 1 to 3, **characterized in that** the sulphonic polyester is obtained from isophthalic acid and from the sodium salt of sulphoisophthalic acid, as the only dicarboxylic acids, from diethylene glycol and from one other diol.

5. Aerosol device according to Claim 4, **characterized in that** the sulphonic polyester is obtained from isophthalic acid and from the sodium salt of sulphoisophthalic acid as the only dicarboxylic acids, from diethylene glycol and from 1,4-cyclohexanemethanol.

6. Aerosol device according to any one of Claims 1 to 5, **characterized in that** the non-associative polyurethane is a dimethylolpropionic acid/isophorone diisocyanate/neopentyl glycol/polyesterdiol/silicone diamine copolymer.

7. Aerosol device according to any one of Claims 1 to 6, **characterized in that** the non-associative polyurethane is present in an amount of from 0.05% to 20% by weight and preferably from 0.10% to 10% by weight relative to the total weight of the composition.

8. Aerosol device according to any one of Claims 1 to 7, **characterized in that** the sulphonic polyester is present in an amount of from 0.1% to 40% by weight, preferably from 1% to 30% and more particularly from 5% to 25% by weight relative to the total weight of the composition.

9. Aerosol device according to any one of the preceding claims, **characterized in that** the cosmetically acceptable aqueous medium consists solely of water or of a mixture of water and of one or more cosmetically acceptable solvents.

10. Aerosol device according to Claim 9, **characterized in that** the cosmetically acceptable solvent is chosen from C₁-C₄ lower alcohols, alkylene polyols and polyol ethers, and mixtures thereof.

11. Aerosol device according to Claim 9 or 10, **characterized in that** the cosmetically acceptable solvent is chosen from ethanol, isopropanol, tert-butanol, n-butanol and propylene glycol.

12. Aerosol device according to Claim 11, **characterized in that** the cosmetically acceptable solvent is ethanol.

13. Aerosol device according to any one of the preceding claims, **characterized in that** water is present in an amount ranging between 15% and 95% and preferably between 20% and 90% by weight relative to the total weight of the composition.

14. Aerosol device according to any one of the preceding claims, **characterized in that** the composition also comprises at least one additive chosen from other cationic, amphoteric, zwitterionic, anionic or nonionic polymers different from those such as defined in any one of Claims 1 to 13, thickeners, plasticizers, nacreous agents, opacifiers, UV-screening agents, sugars, fragrances, mineral, plant and/or synthetic oils, fatty acid esters, dyes, volatile or nonvolatile, organomodified or non-organomodified, cyclic or acyclic, branched or unbranched silicones, mineral or organic, natural or synthetic particles, preserving agents and pH stabilizers.

15. Aerosol device according to any one of the preceding claims, **characterized in that** the composition is in the form of a mousse, a gel, a spray or a lacquer.

16. Aerosol device according to any one of the preceding claims, **characterized in that** the propellant is chosen from hydrocarbon-based gases, fluorinated gases, nitrogen, air, carbon dioxide and dimethyl ether, and mixtures thereof.

17. Aerosol device according to Claim 16, **characterized in that** the propellant is chosen from dimethyl ether and hydrocarbon-based gases, and mixtures thereof.

18. Aerosol device according to any one of Claims 1 to 17, **characterized in that** the propellant is present in an amount of from 5% to 80% by weight and preferably from 5% to 60% by weight relative to the total weight of the composition.

19. Use of the cosmetic composition for treating keratin materials packaged in an aerosol device as defined in the preceding claims, for shaping and/or holding the hairstyle.

20. Cosmetic process for treating the hair, **characterized in that** the cosmetic treatment composition packaged in the aerosol device according to any one of Claims 1 to 18 is applied to the hair.

## Patentansprüche

1. Aerosolvorrichtung, die eine Zusammensetzung für die kosmetische Behandlung von Keratinsubstanzen enthält, die in einem kosmetisch akzeptablen wässrigen Medium mindestens einen in Wasser dispergierbaren Sulfopolyester, der ausgehend von Isophthalsäure gegebenenfalls in der Form des Esters und Sulfoisophthalsäure gegebenenfalls in der Form des Salzes als einzige enthaltenen Dicarbonsäuren, und Diethylenglycol gebildet wird, mindestens ein nicht assoziatives Polyurethan und mindestens ein Treibmittel enthält, wobei das nicht assoziative Polyurethan eine wiederkehrende Grundeinheit der folgenden allgemeinen Formel (I):
-O-B-O-CO-NH-R-NH-CO- (I)
worin:
- B eine zweiwertige C₁-₃₀-Kohlenwasserstoffgruppe ist, wobei diese Gruppe gegebenenfalls mit einer Gruppe substituiert ist, die eine oder mehrere Carbonsäurefunktionen und/oder eine oder mehrere Sulfonsäurefunktionen aufweist, wobei die Carbonsäure- und/oder Sulfonsäurefunktionen in freier Form oder auch teilweise oder vollständig durch eine anorganische oder organische Base neutralisiert vorliegen, und
- R eine zweiwertige Gruppe ist, die unter den aliphatischen Kohlenwasserstoffgruppen mit 1 bis 20 Kohlenstoffatomen, cycloaliphatischen Kohlenwasserstoffgruppen mit 3 bis 20 Kohlenstoffatomen und aromatischen Kohlenwasserstoffgruppen mit 6 bis 20 Kohlenstoffatomen oder deren Kombinationen ausgewählt ist, wobei diese Gruppen substituiert sind oder unsubstituiert vorliegen;
und ferner eine wiederkehrende Grundeinheit der allgemeinen Formel (II) enthält:
-O-P-O-CO-NH-R-NH-CO- (II)
worin:
- P für ein Polysiloxansegment steht und
- R eine zweiwertige Gruppe ist, die unter den aliphatischen Kohlenwasserstoffgruppen mit 1 bis 20 Kohlenstoffatomen, cycloaliphatischen Kohlenwasserstoffgruppen mit 3 bis 20 Kohlenstoffatomen und aromatischen Kohlenwasserstoffgruppen mit 6 bis 20 Kohlenstoffatomen oder deren Kombinationen ausgewählt ist, wobei diese Gruppen substituiert sind oder unsubstituiert vorliegen.

2. Aerosolvorrichtung nach Anspruch 1, **dadurch gekennzeichnet, dass** der Sulfopolyester ausgehend von Isophthalsäure oder einem ihrer Ester, einem Salz von Sulfoisophthalsäure als einzige Dicarbonsäuren, und Diethylenglycol gebildet wird.

3. Aerosolvorrichtung nach Anspruch 2, **dadurch gekennzeichnet, dass** der Sulfopolyester ausgehend von Isophthalsäure, dem Natriumsalz von Sulfoisophthalsäure als einzige Dicarbonsäuren, und Diethylenglycol gebildet wird.

4. Aerosolvorrichtung nach einem der Ansprüche 1 bis 3, **dadurch gekennzeichnet, dass** der Sulfopolyester ausgehend von Isophthalsäure, dem Natriumsalz von Sulfoisophthalsäure als einzige Dicarbonsäuren, Diethylenglycol und einem weiteren Diol gebildet wird.

5. Aerosolvorrichtung nach Anspruch 4, **dadurch gekennzeichnet, dass** der Sulfopolyester ausgehend von Isophthalsäure, dem Natriumsalz von Sulfoisophthalsäure als einzige Dicarbonsäuren, Diethylenglycol und 1,4-Cyclohexanmethanol gebildet wird.

6. Aerosolvorrichtung nach einem der Ansprüche 1 bis 5, **dadurch gekennzeichnet, dass** das nicht assoziative Polyurethan ein Dimethylolpropionsäure/Isophorondiisocyanat/Neopentylglycol/Polyesterdiole/siliconiertes Diamin-Copolymer ist.

7. Aerosolvorrichtung nach einem der Ansprüche 1 bis 6, **dadurch gekennzeichnet, dass** das nicht assoziative Polyurethan in einem Mengenanteil im Bereich von 0,05 bis 20 Gew.-% und vorzugsweise 0,10 bis 10 Gew.-%, bezogen auf das Gesamtgewicht der Zusammensetzung, enthalten ist.

8. Aerosolvorrichtung nach einem der Ansprüche 1 bis 7, **dadurch gekennzeichnet, dass** der Sulfopolyester in einem Mengenanteil von 0,1 bis 40 Gew.-%, vorzugsweise 1 bis 30 Gew.-% und insbesondere 5 bis 25 Gew.-%, bezogen auf das Gesamtgewicht der Zusammensetzung, vorliegt.

9. Aerosolvorrichtung nach einem der vorhergehenden Ansprüche, **dadurch gekennzeichnet, dass** das kosmetisch annehmbare wässrige Medium einzig und allein aus Wasser oder einem Gemisch von Wasser und einem oder mehreren kosmetisch akzeptablen Lösungsmitteln besteht.

10. Aerosolvorrichtung nach Anspruch 9, **dadurch gekennzeichnet, dass** das kosmetisch annehmbare Lösungsmittel unter den niederen Alkoholen mit 1 bis 4 Kohlenstoffatomen, Alkylenpolyolen, Polyolethern und Gemischen davon ausgewählt ist.

11. Aerosolvorrichtung nach Anspruch 9 oder 10, **dadurch gekennzeichnet, dass** das kosmetisch annehmbare Lösungsmittel unter Ethanol, Isopropanol, *tert*-Butanol, n-Butanol und Propylenglycol ausgewählt ist.

12. Aerosolvorrichtung nach Anspruch 11, **dadurch gekennzeichnet, dass** das kosmetisch annehmbare Lösungsmittel das Ethanol ist.

13. Aerosolvorrichtung nach einem der vorhergehenden Ansprüche, **dadurch gekennzeichnet, dass** das Wasser in einer Menge von 15 bis 95 Gew.-% und vorzugsweise 20 bis 90 Gew.-%, bezogen auf das Gesamtgewicht der Zusammensetzung, vorliegt.

14. Aerosolvorrichtung nach einem der vorhergehenden Ansprüche, **dadurch gekennzeichnet, dass** sie ferner mindestens einen Zusatzstoff enthält, der unter anderen kationischen, amphoteren, zwitterionischen, anionischen oder nichtionischen Polymeren, die von den in einem der Ansprüche 1 bis 13 definierten Polymeren verschieden sind, Verdickungsmitteln, Weichmachern, Perlglanzstoffen, Trübungsmitteln, UV-Filtern, Zuckern, Duftstoffen, Mineralölen, pflanzlichen Ölen und/oder synthetischen Ölen, Fettsäureestern, Farbmitteln, flüchtigen oder nichtflüchtigen, gegebenenfalls organomodifizierten, cyclischen oder acyclischen, gegebenenfalls verzweigten Siliconen, natürlichen oder synthetischen, anorganischen oder organischen Partikeln, Konservierungsmitteln und pH-Stabilisatoren ausgewählt ist.

15. Aerosolvorrichtung nach einem der vorhergehenden Ansprüche, **dadurch gekennzeichnet, dass** die Zusammensetzung als Schaum, Gel, Spray oder Lack vorliegt.

16. Aerosolvorrichtung nach einem der vorhergehenden Ansprüche, **dadurch gekennzeichnet, dass** das Treibmittel unter Kohlenwasserstoffgasen, fluorhaltigen Gasen, Stickstoff, Luft, Kohlendioxid, Dimethylether und Gemischen davon ausgewählt ist.

17. Aerosolvorrichtung nach Anspruch 16, **dadurch gekennzeichnet, dass** das Treibmittel unter Dimethylether, Kohlenwasserstoffgasen und Gemischen davon ausgewählt ist.

18. Aerosolvorrichtung nach einem der Ansprüche 1 bis 17, **dadurch gekennzeichnet, dass** das Treibmittel in einer Menge von 5 bis 80 Gew.-% und vorzugsweise von 5 bis 60 Gew.-%, bezogen auf das Gesamtgewicht der Zusammensetzung, vorliegt.

19. Verwendung der in einer wie in den vorhergehenden Ansprüchen definierten Aerosolvorrichtung konfektionierten Zusammensetzung für die kosmetische Behandlung von Keratinsubstanzen für die Formgebung und/oder Festigung der Frisur.

20. Verfahren zur kosmetischen Behandlung der Haare, **dadurch gekennzeichnet, dass** die in der Aerosolvorrichtung nach einem der Ansprüche 1 bis 18 konfektionierte Zusammensetzung für die kosmetische Behandlung auf die Haare aufgetragen wird.
